# EUROPEAN PATENT APPLICATION

(11) **EP 4 506 014 A1**
(43) Date of publication of application: **12.02.2025**
(21) Application number: 24193331.6
(22) Date of filing: 07.08.2024
(51) Int. Cl.: A61K 36/185, A61K 45/06, A61K 31/5513, A61K 31/551, A61K 31/55, A61P 25/08

(54) **PHARMACEUTICAL COMBINATION FOR TREATING OR PREVENTING EPILEPSY AND THE USE THEREOF**

(30) Priority: 09.08.2023 US 202318446761
(71) Applicant: Fu Jen Catholic University, New Taipei City 242062 (TW)
(72) Inventor: WANG, Su-Jane, 242062 New Taipei City (TW); HUNG, Chi-Feng, 242062 New Taipei City (TW)
(74) Representative: Stöckeler, Ferdinand

(57) **Abstract**

The present disclosure provides a birch (*Betula platyphylla*) sap extract combination for treating or preventing epilepsy and use in the preparation a medication. The medication may further include other medications or ingredients to enhance the anti-epileptic effects. The combination of the present disclosure has the characteristics of high efficiency, safety, and convenience, and can effectively treat or prevent epilepsy while reducing medication side effects. The present disclosure also provides a method of treating or preventing epilepsy with the combination.

## Description

### BACKGROUND

### Technology Field

The present disclosure relates to a pharmaceutical combination for treating or preventing epilepsy and the use thereof. In particular, the present disclosure relates to a pharmaceutical combination comprising birch sap extract and at least one other medicament and uses thereof.

### Description of Related Art

The background description includes information that may be useful in understanding the present invention. It is not an admission that any of the information provided herein is prior art or relevant to the presently claimed invention, or that any publication specifically or implicitly referenced is prior art.

Epilepsy is a chronic neurological disease. It is the fourth most common neurological disease after stroke, migraine and Alzheimer's disease, affecting as many as 70 million people worldwide. This disorder is characterized by abnormal firing of nerve cells in the brain, mainly in the cerebral cortex and hippocampus, leading to recurrent, spontaneous, and unpredictable seizures. The pathogenesis of epilepsy may be related to congenital or acquired encephalopathy, such as intracerebral trauma, infection, stroke and tumors. However, about 70% of epilepsy patients cannot find any cause of the disease. Although the cause of most epilepsy is unknown, it is generally believed that nerve cells may be overexcited due to an imbalance between the inhibitory nervous systems (i.e., the GABGA system) and excitatory nervous systems (i.e., the glutamine system) in the brain.

The mechanism of action of antiepileptic drugs currently used to control epileptic seizures is mainly to directly inhibit the excitability of nerve cells by blocking sodium ion channels, such as phenytoin, carbamazepine; inhibit glutamate, such as lamotrigine, felbamate; enhance GABA, such as benzodiazepines, tiagabine, vigabatrin, to regulate neuronal stability. However, although nearly thirty drugs are clinically used to treat epilepsy, approximately one-third of people with epilepsy are resistant to existing drugs. In addition, drug side effects also limit patient compliance with existing medications.

Currently known antiepileptic drugs commonly used have many side effects. For example, the possible side effects marked on the package insert of carbamazepine are hypersomnia, upset stomach, diarrhea, giddiness, head heaviness, ataxia, aleukemia, aplastic anemia, hepatic insufficiency, arrhythmia, eruption, etc. The possible side effects marked on the package insert of phenytoin are nausea, vomiting, pyrosis, loss of appetite, diplopia, nystagmns, ataxia, gingival hyperplasia, hypertrichosis, rough face, athetosis, lymphadenopathy, monster, eruption, etc. Once side effects occur, the treatment usually involves changing the drug, reducing the dose or increasing other drugs to suppress the side effects. However, reducing the dose can easily lead to epileptic recurrence, resulting in psychosomatic suffering for epileptic patients.

Therefore, finding and developing more effective and safer new drugs is necessary for the treatment of epilepsy. Medicinal plants have attracted attention for their wide use in traditional and folk medicine for the treatment and prevention of different neurological disorders, including epilepsy. In recent years, many plant-derived bioactive molecules have been confirmed to produce antiepileptic effects by affecting ion channels, GABA or glutamic acid, such as, flavonoids, alkaloids, terpenoids and cannabidiol. Therefore, medicinal plants have been the target of antiepileptic drug research.

Birch (*Betula platyphylla*) belongs to Betulaceae, Betula genus, distributed in Siberia, Finland, Hokkaido, Mongolia and other places. In the Herbal Foundation Compendium, birch bark has been documented to treat arthritis, skin diseases, physical trauma, and to clear heat and resolve toxins. Currently, birch has been widely used in folk medicine to treat a variety of inflammatory diseases, including hepatitis, acute tonsillitis, pneumonia, chronic bronchitis, cholecystitis, urinary tract infection and burns. Studies have shown that birch has many biological activities, such as anti-inflammatory, anti-oxidant, anti-viral, anti-cancer, anti-fatigue and neuroprotective effects. These activities are related to birch's main active ingredient triterpenes - betulin. However, there are few relevant studies on birch in the central nervous system. Therefore, there is still a need for a pharmaceutical combination containing birch extract to treat or prevent epilepsy.

### SUMMARY

The present disclosure provide a birch sap extract for use in the treatment or prevention of epilepsy.

In one embodiment, the birch sap extract of the present disclosure is obtained from the birch trees grown in Hokkaido, Japan.

In one embodiment, epilepsy is caused by damage to nerve cells in the brain. In another embodiment, epilepsy is caused by the activation of glial cells. In other embodiment, epilepsy is caused by an inflammatory response in the brain caused by inflammatory molecules. In other embodiment, the inflammatory molecule is selected from the group consisting of 1interleukin-1β (IL-1β), interleukin-6 (IL-6), tumor necrosis factor-α (TNF-α), high mobility group Box 1 (HMGB1), interleukin-1 receptor 1 (IL-1R1), and Toll-like receptor-4 (TLR-4).

In one embodiment, the birch sap extract of the present disclosure is further combined with at least one other antiepileptic drug for use in the treatment or prevention of epilepsy. In another embodiment, the at least one other antiepileptic drug is a western medicine. In other embodiment, the at least one other antiepileptic drug is selected from the group of the western medicine consisting of phenytoin, carbamazepine, lamotrigine, felbamate, benzodiazepines, tiagabine, and vigabatrin. In other embodiment, the at least one other antiepileptic drug is a Chinese traditional medical preparation or formula. In other embodiment, the at least one other antiepileptic drug is selected from the group of the Chinese traditional medical preparation or formula consisting of Tall Gaxtraodia Tuber (Gastrodiae Rhizoma), Gambir Plant (Uncariae Ramulus cum Uncis), Sea-ear Shell (Haliotidis Concha), Zhengan Xifeng Decoction, and Gastrodia and Uncaria Decoction.

The present disclosure also provides a method for preparing a birch sap extract, comprising:
selecting mature birch trees and cutting the bark to promote the flow of sap;
collecting the outflowing sap in a collector;
filtering the sap at room temperature to remove impurities; and
removing most of the water in the sap with low-temperature concentration technology to obtain high-concentration birch sap.
The above steps can be increased or decreased according to the extraction of specific components or other actual needs.

In one embodiment, the birch tree is a birch tree grown in Hokkaido, Japan. In another embodiment, the birch sap is harvested in winter or spring. In another embodiment, the birch sap is harvested in early spring.

The present disclosure also provides a pharmaceutical combination for treating or preventing epilepsy comprising birch sap extract and at least one other antiepileptic drug and a pharmaceutically acceptable carrier and/or stabilizer, wherein the amount of the at least one other antiepileptic drug contented in one dose of the pharmaceutical combination is lower than the amount of one dose of the at least one other antiepileptic drug when it is administered alone for treating or preventing epilepsy. In one embodiment, the at least one other antiepileptic drug is selected from the group consisting of phenytoin, carbamazepine, lamotrigine, felbamate, benzodiazepines, tiagabine, and vigabatrin. In one embodiment, the birch sap extract and the at least one other antiepileptic drug are administered to a patient simultaneously, sequentially or at intervals.

Various objects, features, aspects and advantages of the inventive subject matter will become more apparent from the following detailed description of preferred embodiments.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
Figures 1A-1D show the chemical properties of birch sap extract (HCF02), wherein Figures 1A and 1B show the composition of monosaccharides; Figure 1C shows the molecular weight distribution; and Figure 1D shows the ¹H NMR spectrum.
Figures 2A and 2B are histograms of reducing the epileptic behavior of KA-induced animals by pretreatment with birch sap extract. Data are means ± SEM (n = 2-13 rats/group). *, p < 0.05 compared with KA group. ***, p < 0.001 compared with KA group.
Figures 3A and 3B show that birch sap extract and betulin pretreatment prevent KA-induced neuronal cell death in the cerebral cortex and hippocampus. Data are means ± SEM. *, p < 0.05 compared with the control group. ***, p < 0.001 compared with the control group. #, p < 0.05 compared with KA group. n = 3 per group.
Figures 4A and 4B show that birch sap extract and betulin pretreatment reduce KA-induced neuronal cell degeneration in the cerebral cortex and hippocampus. Data are means ± SEM. ***, p < 0.001 compared with the control group. #, p < 0.05 compared with KA group. n = 3 per group.

### DETAILED DESCRIPTION OF THE DISCLOSURE

The following description includes information that may be useful in understanding the present invention. It is not an admission that any of the information provided herein is prior art or relevant to the presently claimed invention, or that any publication specifically or implicitly referenced is prior art.

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details.

### Definitions

As used herein, each of the following terms has the meaning associated with it in this section. It is also to be understood that the terminology used herein is for the purpose of describing particular embodiments of this disclosure only and is not intended to be limiting.

Unless defined otherwise, all technical and scientific terms used herein generally have the same meaning as commonly understood by one of ordinary skill in the art to which this invention belongs. Generally, the nomenclature used herein and the laboratory procedures in animal pharmacology, pharmaceutical science, separation science, and organic chemistry are those well-known and commonly employed in the art. In the methods described herein, the acts can be carried out in any order, except when a temporal or operational sequence is explicitly recited. Furthermore, specified acts can be carried out concurrently unless explicit claim language recites that they be carried out separately. For example, a claimed act of doing X and a claimed act of doing Y can be conducted simultaneously within a single operation, and the resulting process will fall within the literal scope of the claimed process.

The following non-limiting abbreviations are used herein: KA: kainic acid; IL-1β: interleukin-1β; IL-6: interleukin-6; TNF-α: tumor necrosis factor-α; HMGB1: high mobility group Box 1; IL-1R1: interleukin-1 receptor 1; CBZ: carbamazepine; CABA: gamma-aminobutyric acid; and TLR-4: Toll-like receptor-4.

As used in this specification and the appended claims, the singular forms "a", "an" and "the" include plural referents unless the content clearly dictates otherwise. By way of example, "a component" means one component or more than one component.

As used herein, the term "about" will be understood by persons of ordinary skill in the art and will vary to some extent on the context in which it is used. As used herein, "about" when referring to a measurable value such as an amount, a temporal duration, and the like, is meant to encompass variations of ±20%, ±10%, ±5%, ±1%, or ±0.1% from the specified value, as such variations are appropriate to perform the disclosed methods. Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. The numerical values presented in some embodiments of the invention may contain certain errors necessarily resulting from the standard deviation found in their respective testing measurements.

In one aspect, the terms "co-administered" and "co-administration" as relating to a subject refer to administering to the subject a birch sap extract of the present disclosure along with other medicaments that may also treat or prevent a disease or disorder contemplated herein. In certain embodiments, the co-administered birch sap extract and other medicaments are administered separately, or in any kind of combination as part of a single therapeutic approach.

As used herein, a "disease" is a state of health of a subject wherein the subject cannot maintain homeostasis, and wherein if the disease is not ameliorated then the subject's health continues to deteriorate.

As used herein, a "disorder" in a subject is a state of health in which the subject is able to maintain homeostasis, but in which the subject's state of health is less favorable than it would be in the absence of the disorder. Left untreated, a disorder does not necessarily cause a further decrease in the subject's state of health.

As used herein, the term "pharmaceutically acceptable" refers to a material, such as a carrier or diluent, which does not abrogate the biological activities or properties of the compounds or pharmaceutical ingredients useful within the present disclosure, and is relatively non-toxic, i.e., the material may be administered to a subject without causing undesirable biological effects or interacting in a deleterious manner with any of the components of the pharmaceutical combination in which it is contained.

As used herein, a "pharmaceutically effective amount," "therapeutically effective amount," or "effective amount" of a compound or ingredient in the pharmaceutical combination is that amount of compound that is sufficient to provide a beneficial effect to the subject to which the compound is administered.

As used herein, the terms "subject", "individual" and "patient" can be used interchangeably, and may refer to a human or non-human mammal. Non-human mammals include, for example, livestock and pets, such as ovine, porcine, canine, feline and murine mammals. In certain embodiments, the subject is human.

The term "prevent," "preventing," or "prevention" as used herein means avoiding or delaying the onset of symptoms associated with a disease or condition in a subject that has not developed such symptoms at the time the administering of an agent or a pharmaceutical combination commences.

The terms "treat," "treating" and "treatment," as used herein, means reducing the frequency or severity with which symptoms of a disease or condition are experienced by a subject by virtue of administering an agent or a pharmaceutical combination to the subject.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual and partial numbers within that range, for example, 1, 2, 2.7, 3, 4, 5, 5.3, and 6. This applies regardless of the breadth of the range.

### Birch sap

The birch sap of the present disclosure is a colorless or light yellow transparent liquid collected and/or extracted from birch bark and/or branches. Birch sap contains many active substances, such as betulin, polysaccharides, saponins, proteins, and minerals, and has high medicinal value. In traditional Chinese medicine theory, birch sap is classified as a heat-clearing and detoxicating medicine. The currently disclosed curative effects and benefits are as follows:
Heat-clearing and detoxicating: birch sap has heat-clearing and detoxicating effects, and can help remove heat and dampness in the body, relieve fever, thirst, sore throat, rash and other symptoms.
Inducing diuresis for removing edema: birch sap can promote the excretion function of the kidneys, increase urine output, help eliminate excess water in the body, and relieve edema and other symptoms.
Anti-inflammatory and analgesia: birch sap contains some anti-inflammatory and pain-relieving ingredients, such as menthol and salicylic acid, and can help relieve pain and inflammation.
Improving digestion: birch sap can promote gastrointestinal peristalsis, increase the secretion of digestive fluid, and improve gastrointestinal problems, such as indigestion and diarrhea.
Nutritional supplement: birch sap contains a variety of nutrients, such as vitamin C, vitamin B complex, iron, potassium, etc., and can help supplement the nutrients needed by the body and enhance the body's immunity and disease resistance.

In one embodiment, the birch sap of the present disclosure is harvested in winter or spring. Preferably, the birch sap is harvested in early spring. In another embodiment, the birch sap of the present disclosure is harvested from birch trees that are 20 to 50 years old. In other embodiment, the birch trees are the birch trees grown in Hokkaido, Japan.

### Combination therapies

In one aspect, the birch sap extract of the present disclosure are useful in combination with one or more additional agents useful for treating or preventing epilepsy. These additional agents may comprise compounds (e.g., commercially available compounds) or pharmaceutical ingredients known to treat, prevent, or reduce the symptoms of epilepsy.

In one embodiment, the present disclosure provides a method of treating or preventing epilepsy in a subject. The method comprises administering to the subject a therapeutically effective amount of the birch sap extract of the present disclosure. In one embodiment, the subject is further co-administered with at least one other agent that aids in the treatment or prevention of epilepsy. In another embodiment, the at least one other agent is a Chinese traditional medical preparation or formula, such as Tall Gaxtraodia Tuber (Gastrodiae Rhizoma), Gambir Plant (Uncariae Ramulus cum Uncis), Sea-ear Shell (Haliotidis Concha), Zhengan Xifeng Decoction, Gastrodia and Uncaria Decoction and the like, wherein the dosage of the Chinese traditional medical preparation can be adjusted by a Chinese medicine doctor according to the conventional dosage. In other embodiment, the at least one other agent is a Chinese traditional medical preparation or formula, and it is formulated together with birch sap extract. In other embodiment, the at least one other antiepileptic drug is a western medicine, such as phenytoin, carbamazepine, lamotrigine, felbamate, benzodiazepines, tiagabine, and vigabatrin, wherein the dose of the western medicine can be adjusted by a doctor according to the recommended dosage of each medicine.

In one embodiment, the subject suffering from epilepsy may be a subject who has been diagnosed with epilepsy for the first time, or who is taking at least one antiepileptic drug. In one embodiment, a combination of birch sap extract and at least one antiepileptic drug may be provided to the subject diagnosed with epilepsy for the first time, wherein the at least one antiepileptic drug is administered in combination at a lower dose than when administered alone. In another embodiment, the birch sap extract can further be provided to the subject who is taking at least one antiepileptic drug, and the dose of the at least one antiepileptic drug can be reduced.

### Administration/Dosing

The regimen of administration may affect what constitutes an effective amount. The pharmaceutical combination may be administered to the patient either prior to or after the onset of a disease or disorder. Further, several divided dosages, as well as staggered dosages may be administered daily or sequentially. Further, the dosages of ingredients in the pharmaceutical combination may be proportionally increased or decreased as indicated by the exigencies of the therapeutic or prophylactic situation.

Administration of the birch sap extract of the present disclosure to a patient may be carried out using known procedures, at dosages and for periods of time effective to treat or prevent epilepsy. An effective amount of the birch sap extract necessary to achieve the effect on treating or preventing epilepsy may vary according to factors, such as the activity of the birch sap extract employed; the time of administration; the rate of excretion of the extract; the duration of the treatment; other medicines, compounds or materials used in combination with the extract; the state of the disease or disorder, age, sex, weight, condition, general health and prior medical history of the patient being treated, and like factors well-known in the medical arts. Dosage regimens may be adjusted to provide the optimum therapeutic response. For example, several divided doses may be administered daily or the dose may be proportionally reduced as indicated by the exigencies of the therapeutic situation.

Although the descriptions of the birch sap extract provided herein are principally directed to pharmaceutical combinations suitable for ethical administration to humans, it will be understood by the skilled artisan that such combinations are generally suitable for administration to various animals. Modification of the combination suitable for administration to humans in order to render the combinations suitable for administration to various animals is well understood, and the ordinarily skilled veterinary pharmacologist can design and perform such modification with merely ordinary, if any, experimentation. Subjects to which administration of the birch sap extract or the pharmaceutical combination of the present disclosure is contemplated include, but are not limited to, humans, other primates, and mammals including commercially relevant mammals, such as bovine, porcine, equine, ovine, feline, and canine.

Humans and animals do not have the same tolerate for the same drug. Generally, the tolerance of animals is greater than that of humans. Calculated by unit body weight or unit body surface area, the dose of drugs used by animals is larger than that of humans. The dosage per unit body weight of animals and humans can be calculated by referring to the following general conversion table.

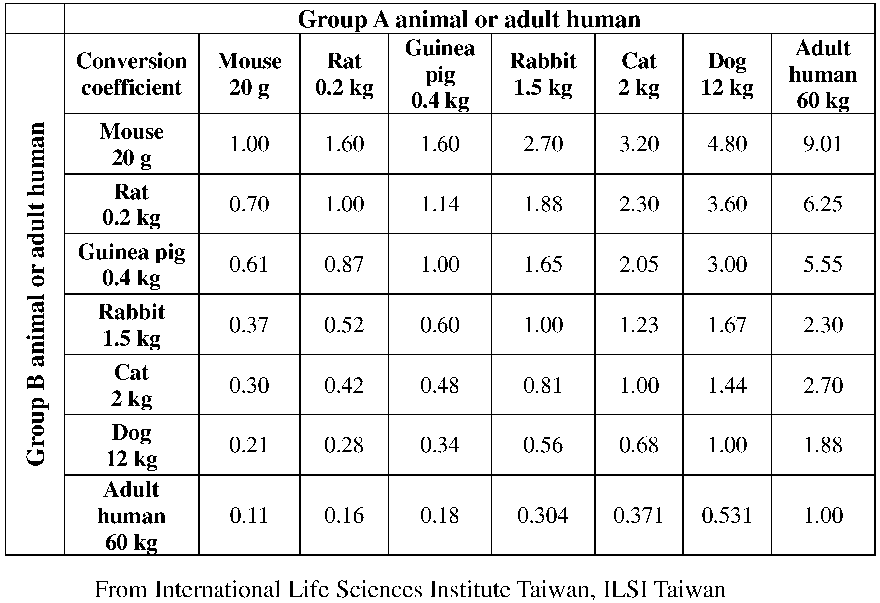

For example, taking the rat model of the embodiment of the present disclosure as an example, the birch sap extract can be administered orally at 3 ~ 20 mg/kg per dose, preferably, 6 ~ 10 mg/kg per dose. For example, the birch sap extract can be administered orally at 6 mg/kg or 10 mg/kg per dose. After conversion with the above unit body weight dose conversion table, the dose for adult human is 0.48 ~ 3.2 mg/kg per dose (that is, 3 x 0.16 ~ 20 x 0.16 mg/kg), preferably, 0.96 - 1.6 mg/kg per dose. The dose of the birch sap extract of the present disclosure suitably for other animals can also be obtained from the above conversion table per unit body weight or other conversion tables per unit body surface area.

In certain embodiments, the birch sap extract or the pharmaceutical combination of the present disclosure are administered to the patient in dosages that range from one to five times per day or more. In other embodiments, the birch sap extract or the pharmaceutical combination of the present disclosure are administered to the patient in range of dosages that include, but are not limited to, once every day, every two days, every three days to once a week, and once every two weeks. It will be readily apparent to one skilled in the art that the frequency of administration of the various combinations of the present disclosure will vary from subject to subject depending on many factors including, but not limited to, age, the severity of disease or disorder to be treated, gender, overall health, and other factors. Thus, the invention should not be construed to be limited to any particular dosage regime and the precise dosage and combination to be administered to any patient will be determined by the attending physician taking all other factors about the patient into account.

In one embodiment, when administering the pharmaceutical combination comprising the birch sap extract of the present disclosure and at least one other antiepileptic drug, the birch sap extract of the present disclosure and the at least one other antiepileptic drug can be administered to the subject at the same time. In another embodiment, the birch sap extract and the at least one other antiepileptic drug are administered to the subject simultaneously, sequentially or at intervals.

### Administration

### Oral Administration

Liquid preparation for oral administration may be in the form of solutions, syrups or suspensions. The liquid preparations may be prepared by conventional means with pharmaceutically acceptable additives, such as vegetable syrup, pharmaceutically acceptable preservatives, etc.

Those skilled in the art will recognize, or be able to ascertain using no more than routine experimentation, numerous equivalents to the specific procedures, embodiments, claims, and examples described herein. Such equivalents were considered to be within the scope of the present disclosure and covered by the claims appended hereto. For example, it should be understood, that modifications in reaction conditions, including but not limited to reaction times, reaction size/volume, and experimental reagents, such as solvents, catalysts, pressures, atmospheric conditions, and reducing/oxidizing agents, with art-recognized alternatives and using no more than routine experimentation, are within the scope of the present disclosure.

It is to be understood that, wherever values and ranges are provided herein, the description in range format is merely for convenience and brevity and should not be construed as an inflexible limitation on the scope of the present disclosure. Accordingly, all values and ranges encompassed by these values and ranges are meant to be encompassed within the scope of the present disclosure. Moreover, all values that fall within these ranges, as well as the upper or lower limits of a range of values, are also contemplated by the present disclosure. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present disclosure. However, they are in no way a limitation of the teachings or disclosure of the present disclosure as set forth herein.

### EXAMPLES

The present disclosure is now described with reference to the following Examples. These Examples are provided for the purpose of illustration only, and the present disclosure is not limited to these Examples, but rather encompasses all variations that are evident as a result of the teachings provided herein.

### Materials & Methods

The following procedures can be utilized in preparing and/or testing exemplary birch sap extract of the present disclosure.

### Birch sap extract

Follow the steps below to extract sap from birch (*Betula platyphylla*) and prepare birch sap extract:

| | |
|---|---|
| Step 1: | selecting mature birch trees and cutting the bark to promote the flow of sap; |
| Step 2: | using a collector to collect the outflowing sap and avoid external contamination; |
| Step 3: | filtering the sap at room temperature to remove impurities; and |
| Step 4: | removing most of the water in the sap with low-temperature concentration technology to obtain high-concentration birch sap extract. |

### Experimental animals

Male Sprague-Dawley rats (SD rat, 150-200 g) were purchased from BioLASCO (Taiwan) and bred in Fu Jen Laboratory Animal Center. All animal experiments were approved by the IACUC of Fu Jen Catholic University. According to the 3R principles, all experiments were performed to minimize animal suffering and use the minimum number of animals necessary to produce reliable results.

### Example 1 Component analysis of birch sap

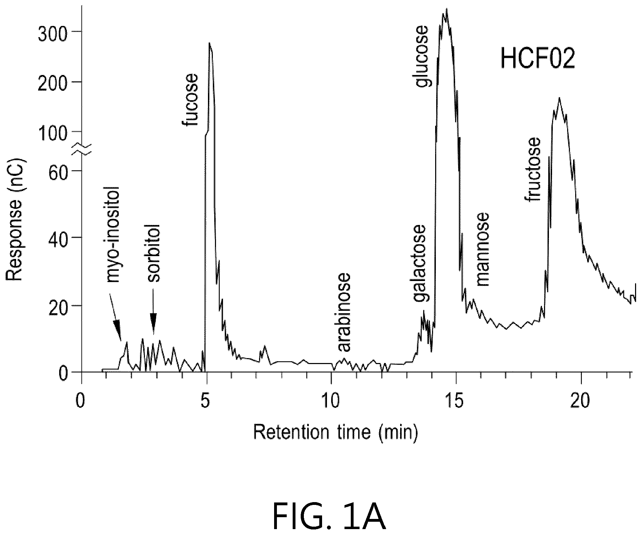

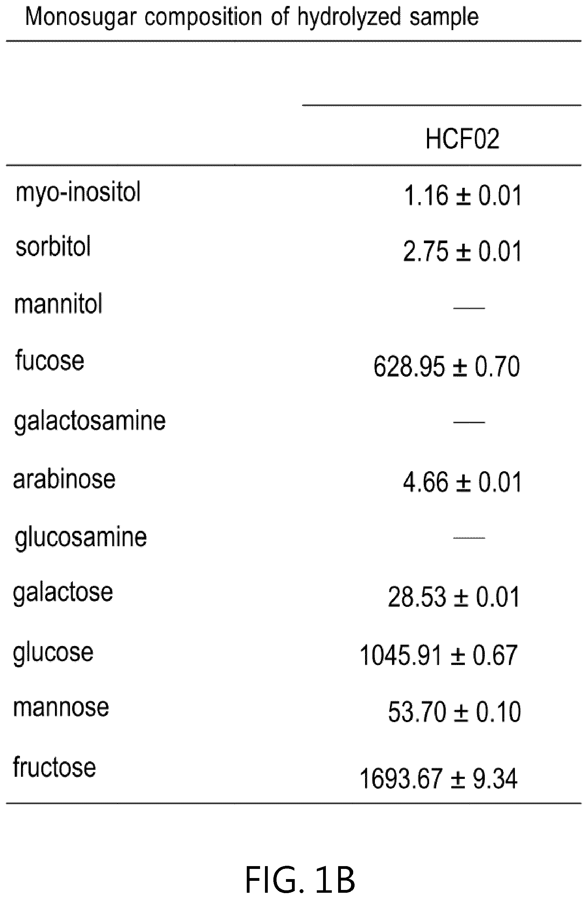

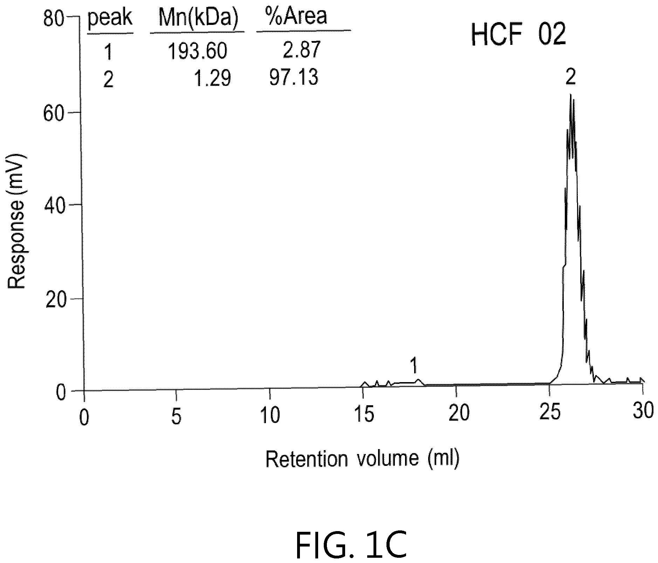

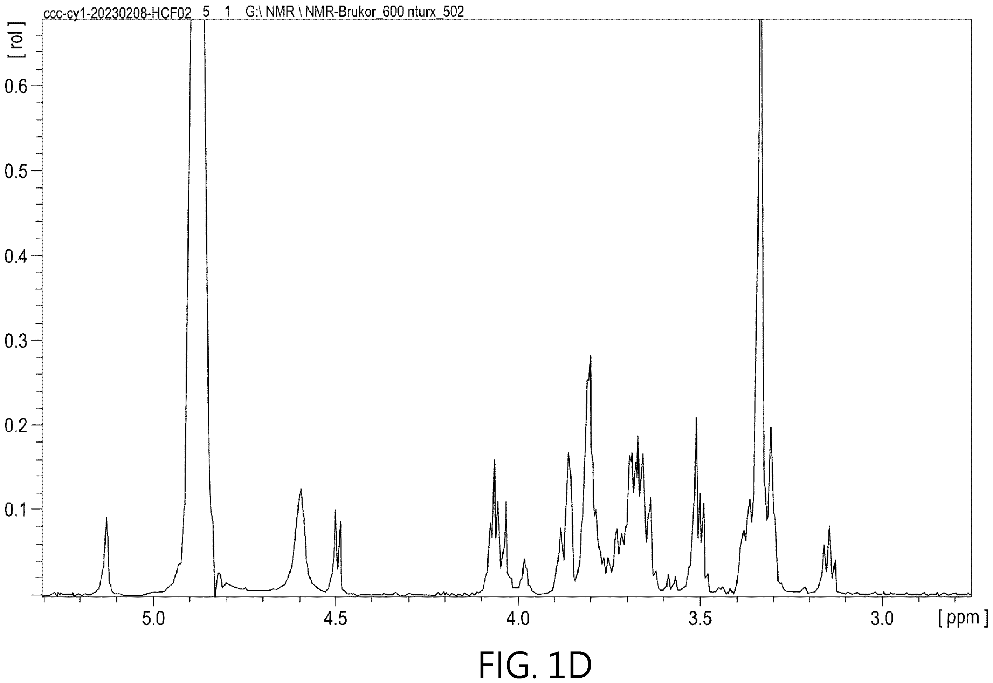

The sap extracted from birch trees was analyzed by monosaccharide analysis. In the monosaccharide analysis of the birch sap, the ratio of the three main monosaccharides, fructose, glucose and fucose, in the birch sap extract was determined to be 27:17:10 (Figs. 1A and 1B). By single-column high pressure size exclusion chromatography (HPSEC) analysis, the molecular weight of the birch sap was about or less than 1.29 kDa (Fig. 1C). The ¹H NMR spectrum of the birch sap (Fig. 1D) showed two anomeric protons at δH 5.13 and δH 4.45. The signals at δH 5.13 and δH 4.45 appear as doublets at a ratio of 100:124, with two monosaccharide residues and α-anomeric configuration (³J_{1,2} = 3.4 Hz) and β-anomeric configuration (³J_{1,2} = 7.7 Hz), respectively. According to the above analysis results, the amount of the birch sap extract used in subsequent biological tests can be further quantified.

### Example 2 KA-induced epilepsy animal model

The antiepileptic effect of the birch sap extract was evaluated using kainic acid (KA)-induced epilepsy model in animals. KA is an analogue of glutamate, and injection of KA into rats will increase the release of glutamate and overactivate glutamate receptors, resulting in progressive limbic seizures in rats. This phenomenon leads to increased calcium ions in nerve cells, oxidative stress, and loss of mitochondrial function, resulting in the death of nerve cells in many areas of the brain, especially in the cerebral cortex and hippocampus. In addition, KA-induced neuronal cell death is related to glial cell activation and inflammatory response caused by inflammatory molecules, such as interleukin-1β (IL-1β), interleukin-6 (IL-6), tumor necrosis factor-α (TNF-α), high mobility group Box 1 (HMGB1), interleukin 1 receptor 1 (IL-1R1), and Toll-like receptor-4 (TLR-4). Since these pathological changes induced by KA are similar to the behavior and pathology of human epilepsy, the KA-induced epilepsy model in animals has been widely used to screen new compounds with antiepileptic activity.

Epileptic behavioral expressions were analyzed within 4 hours after intraperitoneal injection of KA in rats. Seizures were assessed according to the Racine scale (1972), as shown in the table below.

| Seizure stage | Behavioral expression |
|---|---|
| 1 | facial clonus |
| 2 | nodding and wet dog shaking |
| 3 | forelimb clonus |
| 4 | forelimb clonus with rearing |
| 5 | rearing, jumping, and falling |

### Example 3 Evaluation of the effect of birch sap extract on KA-induced epileptic behavior in animals

Sprague-Dawley rats were divided into the following groups according to the administration of different test substances:
1. Negative control group: normal saline
2. KA group: 15 mg/kg/i.p./single dose of kainic acid (KA)
3. Low-dose group of birch sap extract: 6 mg/kg of birch sap extract
4. High-dose group of birch sap extract: 10 mg/kg of birch sap extract
5. High-dose group of antiepileptic drug: 100 mg/kg of carbamazepine (CBZ)
6. Antiepileptic pharmaceutical combination group: 6 mg/kg of birch sap extract and 50 mg/kg of CBZ
7. Low-dose group of antiepileptic drug: 50 mg/kg of CBZ

The control group, birch sap extract groups, antiepileptic drug groups, and antiepileptic pharmaceutical combination group were fed with normal saline, birch sap extract and/or CBZ through feeding tubes at regular intervals every day. One week later, except for the negative control group, rats in each group were intraperitoneally injected with KA (15 mg/kg/i.p./single dose), and the dose of KA injection was based on the previous study. The epileptic behavior of rats was analyzed according to Racine scale (1972) within 4 hours after intraperitoneal injection of KA. The results are shown in Table 1.

**Table 1. The birch sap extract pretreatment relieves KA-induced epileptic behavior in animals.**

| | KA | birch sap extract (6 mg/kg) + KA | birch sap extract (10 mg/kg) + KA | CBZ (100 mg/kg) + KA | birch sap extract (6 mg/kg) + CBZ (50 mg/kg) + KA | CBZ (50 mg/kg) + KA |
|---|---|---|---|---|---|---|
| Seizure stage | 4.6 ± 0.2 | 3.1 ± 0.7* | 1.8 ± 0.4*** | 0.9 ± 0.5*** | 2 ± 1.5** | 3.6 ± 0.3 |
| Death | 2/11 | 0/6 | 2/11 | 1/11 | 0/3 | 0/3 |
| Latency to first seizure (min) | 66.6 ± 6.4 | 159.5 ± 20.5*** | 165.5 ± 14.5*** | 140 | 168 | 89 ± 17 |
| Seizure % | 91% | 33% | 18% | 10% | 33% | 66% |

| | | | | | | |
|---|---|---|---|---|---|---|
| Data are mean ± SEM. **, p < 0.05 compared with KA group. ***, p < 0.001 compared with KA group. | | | | | | |

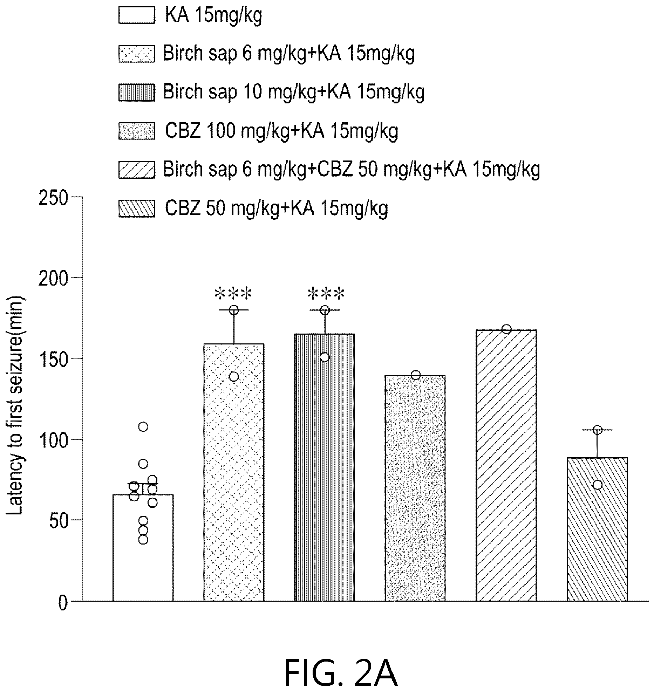

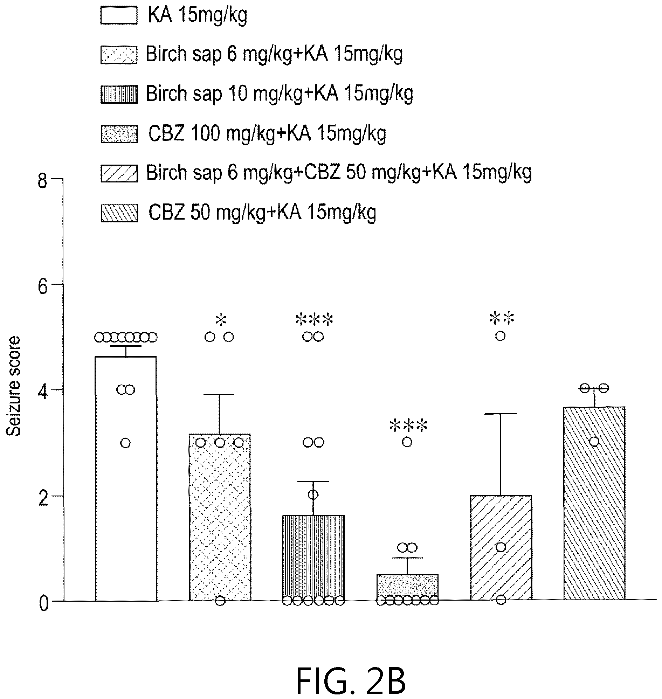

As a result of the experiment, it was found that KA administration induced seizures in 91% of the rats in the KA group (positive control group). Seizure time and stage were 66.6 ± 6.4 minutes and 4.6 ± 0.2, respectively. Compared with the KA group, oral pretreatment with the birch sap extract of the present disclosure for seven days can significantly prolong the time of KA-induced seizures and reduce the severity of KA-induced seizures (p < 0.05; Figs. 2A and 2B). The high-dose group of birch sap extract (10 mg/kg) had a better inhibitory effect on the severity of KA-induced seizures. This effect is similar to the high-dose group of antiepileptic drug (CBZ 100 mg/kg). This result proves that the birch sap extract has the effect of preventing KA-induced seizures. In other words, the birch sap extract of the present disclosure can be used instead of antiepileptic drugs.

In addition, in the low-dose group of antiepileptic drug, the time and severity of seizures were not statistically different from those in the KA group. However, if it is used in combination with low-dose birch sap extract, that is, the antiepileptic pharmaceutical combination group (6 mg/kg of birch sap extract and 50 mg/kg of CBZ), the time and severity of seizures in animals are significantly improved compared with the KA group (p < 0.05 ; Figs. 2A and 2B), which can greatly improve the shortcoming that low-dose antiepileptic drugs cannot effectively suppress the symptoms of epilepsy. Since the antiepileptic pharmaceutical combination group can produce the effect of preventing KA-induced seizures similar to that of the high-dose group of antiepileptic drug, this result shows that the combination of low-dose birch sap extract and low-dose CBZ can be used to prevent KA-induced seizures in animals, and low doses of CBZ can further reduce the side effects of antiepileptic drug.

In this experiment with rats, it is confirmed that the method of the present disclosure can be used to prevent seizures, especially for refractory epileptic symptoms. When the birch sap extract of the present disclosure is used alone, antiepileptic effects comparable to those of antiepileptic drugs (such as CBZ) can be obtained. In other words, the birch sap extract of the present disclosure can be used instead of antiepileptic drugs. In addition, when the birch sap extract is used in combination with antiepileptic drugs (such as CBZ), the antiepileptic effects can still be achieved with further reduction of the dose of antiepileptic drugs. Therefore, whether the birch sap extract of the present disclosure is used alone or in combination with antiepileptic drugs, the side effects of antiepileptic drugs can be reduced. The method provided by the present disclosure has lower toxicity and side effects, making it a safe and effective method for preventing epilepsy.

### Example 4 Histopathological analysis of birch sap extract on cranial nerves

Continuing from Example 3, the experimental animals in each group were sacrificed three days after intraperitoneal injection of KA (15 mg/kg), and the brain tissues were taken out for sectioning and other relevant experimental analysis.

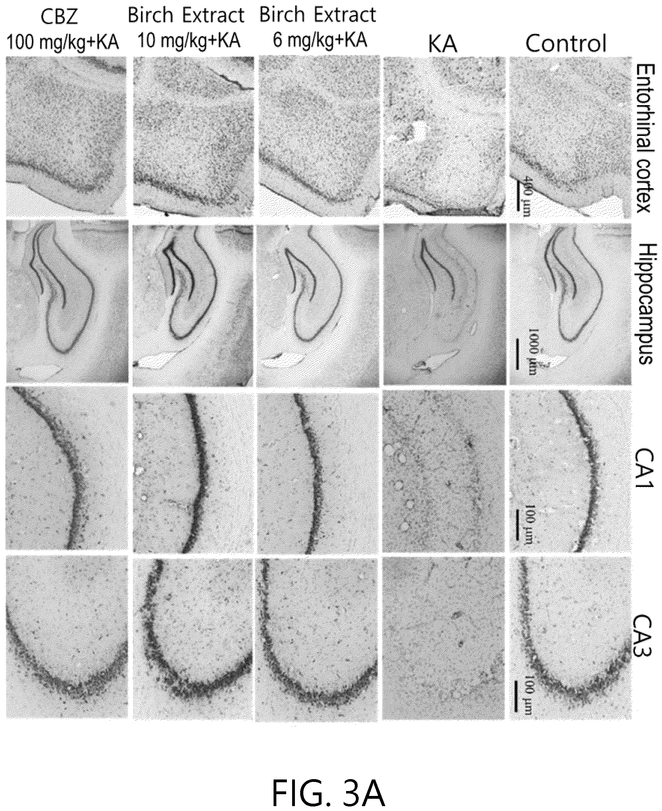

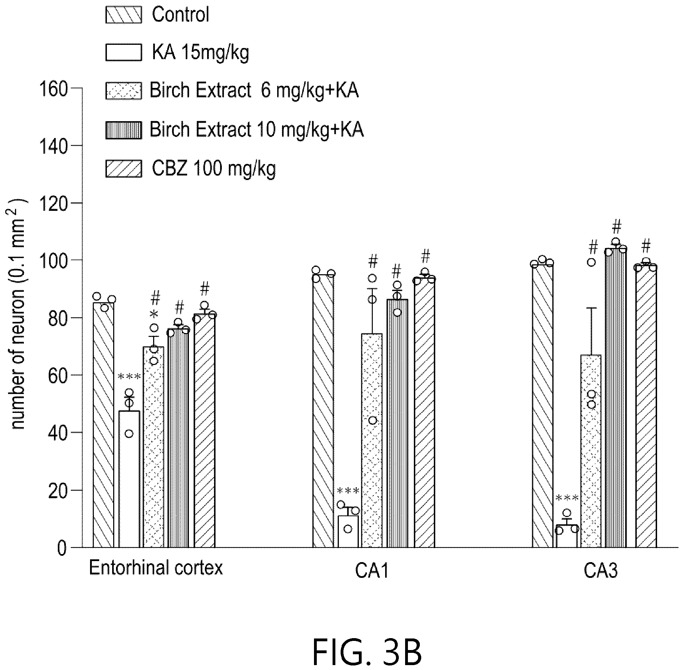

After the brain tissue was stained with Nissl staining, it was found that the brain cerebral cortex and hippocampus CA1/CA3 nerve cells in the KA group were significantly reduced, and the pretreatment with the birch sap extract and betulin can reduce the decrease in the number of neurocytes caused by KA (p < 0.05; Figs. 3A and 3B).

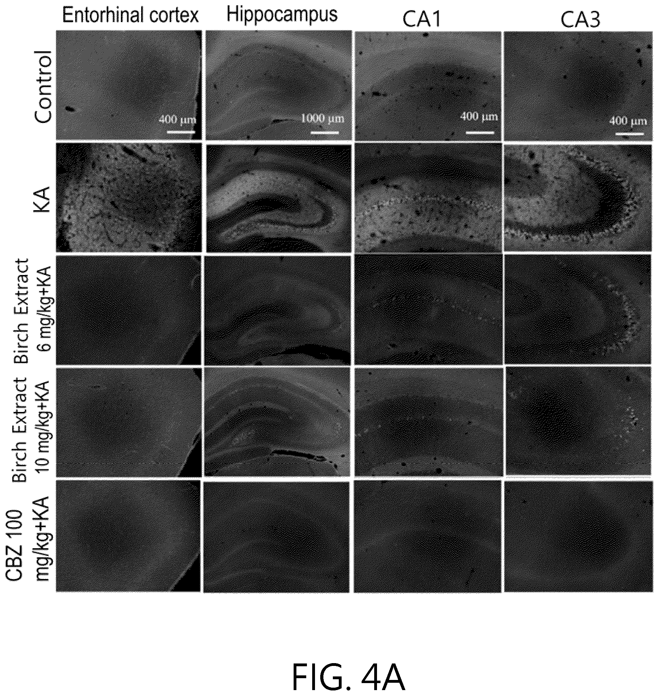

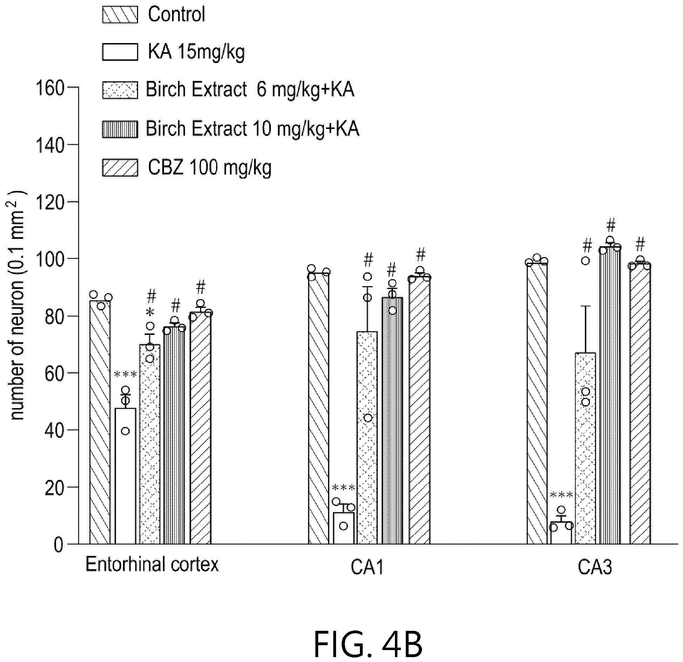

Moreover, in the Fluoro-Jade B (FJB) staining, a phenomenon was also found that the number of CA1/CA3 degenerative cells in the cerebral cortex and hippocampus of the KA group increased. This phenomenon can also be reduced by the pretreatment with the birch sap extract and betulin (p < 0.05; Figs. 4A and 4B). These preliminary results suggest that the pretreatment with the birch sap extract and/or betulin could prevent KA-induced neuronal damage.

The results of the above animal experiments prove that the birch sap extract of the present disclosure can significantly prolong the inhibition time of KA-induced seizure and reduce the severity of KA-induced seizure, which proves that the birch sap extract has the effect of preventing epilepsy. Therefore, birch sap has the prospect of being used in the development of antiepileptic drugs and/or related products.

### Enumerated Embodiments:

The following exemplary embodiments are provided, the numbering of which is not to be construed as designating levels of importance.

Embodiment 1 provides a pharmaceutical combination for use in the treatment or prevention of epilepsy, wherein the pharmaceutical combination comprises birch sap extract and at least one other antiepileptic drug.

Embodiment 2 provides the pharmaceutical combination for use according to Embodiment 1, wherein the birch sap extract is prepared from the birch trees grown in Hokkaido, Japan.

Embodiment 3 provides the pharmaceutical combination for use according to Embodiment 1, wherein the epilepsy is caused by damage to nerve cells in the brain.

Embodiment 4 provides the pharmaceutical combination for use according to Embodiment 1, wherein the epilepsy is caused by the activation of glial cells.

Embodiment 5 provides the pharmaceutical combination for use according to Embodiment 1, wherein the epilepsy is caused by an inflammatory response in the brain caused by inflammatory molecules.

Embodiment 6 provides the pharmaceutical combination for use according to Embodiment 5, wherein the inflammatory molecule is selected from the group consisting of interleukin-1β (IL-1β, interleukin-6 (IL-6), tumor necrosis factor-α (TNF-α), high mobility group Box 1 (HMGB1), interleukin-1 receptor 1 (IL-1R1), and Toll-like receptor-4 (TLR-4).

Embodiment 7 provides the pharmaceutical combination for use according to Embodiment 1, wherein the at least one other antiepileptic drug is a western medicine.

Embodiment 8 provides the pharmaceutical combination for use according to Embodiment 7, wherein the at least one other antiepileptic drug is selected from the group of the western medicine consisting of phenytoin, carbamazepine, lamotrigine, felbamate, benzodiazepines, tiagabine, and vigabatrin.

Embodiment 9 provides the pharmaceutical combination for use according to Embodiment 1, wherein the at least one other antiepileptic drug is a Chinese traditional medical preparation or formula.

Embodiment 10 provides the pharmaceutical combination for use according to Embodiment 9, wherein the at least one other antiepileptic drug is selected from the group of the Chinese traditional medical preparation or formula consisting of Tall Gaxtraodia Tuber (Gastrodiae Rhizoma), Gambir Plant (Uncariae Ramulus cum Uncis), Sea-ear Shell (Haliotidis Concha), zhengan xifeng decoction; zhengan xifeng tang, and Gastrodia and Uncaria Decoction.

Embodiment 11 provides the pharmaceutical combination for use according to of Embodiment 1, wherein the medicament is used to treat or prevent epilepsy in humans.

Embodiment 12 provides the pharmaceutical combination for use according to Embodiment 1, wherein the medicament is used to treat or prevent epilepsy in non-humans mammals.

Embodiment 13 provides a method for preparing a birch sap extract, comprising:
selecting mature birch trees and cutting the bark to promote the flow of sap;
collecting the outflowing sap in a collector;
filtering the sap at room temperature to remove impurities; and
removing most of the water in the sap with low-temperature concentration technology to obtain high-concentration birch sap extract.

Embodiment 14 provides the method according to Embodiment 13, wherein the birch tree is a birch tree grown in Hokkaido, Japan.

Embodiment 15 provides the method according to Embodiment 14, wherein the birch sap is harvested from winter to spring.

Embodiment 16 provides the method according to Embodiment 14, wherein the birch sap is harvested in early spring.

Embodiment 17 provides a pharmaceutical combination for treating or preventing epilepsy comprising the birch sap extract of any one of Embodiments 13-16 and at least one other antiepileptic drug, wherein the amount of the at least one other antiepileptic drug contented in one dose of the pharmaceutical combination is lower than the amount of one dose of the at least one other antiepileptic drug when it is administered alone for treating or preventing epilepsy.

Embodiment 18 provides the pharmaceutical combination according to Embodiment 17, wherein the birch sap extract further comprises a pharmaceutically acceptable carrier and/or stabilizer.

Embodiment 19 provides the pharmaceutical combination according to Embodiment 17, wherein the at least one other antiepileptic drug is selected from the group consisting of phenytoin, carbamazepine, lamotrigine, felbamate, benzodiazepines, tiagabine, and vigabatrin.

Embodiment 20 provides the pharmaceutical combination according to Embodiment 17, wherein the at least one other antiepileptic drug is carbamazepine.

Embodiment 21 provides the pharmaceutical combination according to Embodiment 18, wherein the birch sap extract and the at least one other antiepileptic drug are administered to a subject simultaneously, sequentially or at intervals.

While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A pharmaceutical combination for use in the treatment or prevention of epilepsy comprising a birch (*Betula platyphylla*) sap extract and at least one other antiepileptic drug, wherein the birch sap extract is prepared by filtering and concentrating birch sap at room temperature.

2. The pharmaceutical combination for use according to claim 1, wherein the birch sap is obtained from the birch trees grown in Hokkaido, Japan, from winter to spring.

3. The pharmaceutical combination for use according to claim 1, wherein the epilepsy is caused by damage to nerve cells in the brain.

4. The pharmaceutical combination for use according to claim 1, wherein the epilepsy is caused by the activation of glial cells.

5. The pharmaceutical combination for use according to claim 1, wherein the epilepsy is caused by an inflammatory response in the brain caused by inflammatory molecules.

6. The pharmaceutical combination for use according to claim 5, wherein the inflammatory molecule is selected from the group consisting of interleukin-1β (IL-1β), interleukin-6 (IL-6), tumor necrosis factor-α (TNF-α), high mobility group Box 1 (HMGB1), interleukin-1 receptor 1 (IL-1R1), and Toll-like receptor-4 (TLR-4).

7. The pharmaceutical combination for use according to claim 1, wherein the at least one other antiepileptic drug is selected from the group of consisting of phenytoin, carbamazepine, lamotrigine, felbamate, benzodiazepines, tiagabine, and vigabatrin.

8. The pharmaceutical combination for use according to claim 1, wherein the at least one other antiepileptic drug is carbamazepine.

9. The pharmaceutical combination for use according to claim 1, wherein the birch sap extract and the at least one other antiepileptic drug are administered to a subject simultaneously, sequentially or at intervals.

10. The pharmaceutical combination for use according to claim 9, wherein the subject is a mammal.

11. The pharmaceutical combination for use according to claim 9, wherein the subject is a human.

12. A pharmaceutical combination for treating or preventing epilepsy comprising birch sap extract and at least one other antiepileptic drug, wherein the birch sap extract is prepared by filtering and concentrating birch sap at room temperature, and the amount of the at least one other antiepileptic drug contented in one dose of the pharmaceutical combination is lower than the amount of one dose of the at least one other antiepileptic drug when it is administered alone for treating or preventing epilepsy.

13. The pharmaceutical combination of claim 12, wherein the birch sap is obtained from the birch trees grown in Hokkaido, Japan, from winter to spring.

14. The pharmaceutical combination of claim 12, wherein the at least one other antiepileptic drug is selected from the group consisting of phenytoin, carbamazepine, lamotrigine, felbamate, benzodiazepines, tiagabine, and vigabatrin.

15. The pharmaceutical combination of claim 12, wherein the at least one other antiepileptic drug is carbamazepine.
